# EUROPEAN PATENT APPLICATION

(11) **EP 2 404 578 A1**
(43) Date of publication of application: **11.01.2012**
(21) Application number: 10748831.4
(22) Date of filing: 05.03.2010
(51) Int. Cl.: A61F 5/11

(54) **TOOL FOR CORRECTING INGROWN NAIL**

(30) Priority: 06.03.2009 JP 2009053912; 16.09.2009 JP 2009215006
(71) Applicant: Suzuki, Shunji, Kasaigun, Hokkaido 089-1332 (JP); Ozawa, Tampopo, Imabari-shi, Ehime 794-0063 (JP)
(72) Inventor: SUZUKI, Shunji, Nakasatsunai-mura Kasaigun, Hokkaido 089-1332 (JP)
(74) Representative: Twelmeier Mommer & Partner
(86) International application number: PCT/JP2010/053622
(87) International publication number: WO 2010/101238

(57) **Abstract**

To provide an incurvated nail correction tool for deformed nails that can be put on easily by a patient alone without causing pain, the incurvated nail correction tool 10 includes a central portion 12 made of an elastic wire material bent in a waveform, the waveform being formed, in its free state, within a flat plane, or a curved plane flatter than a curve that runs along a nail plate in contact therewith, U-shaped portions 14 connecting both ends of the central portion 12 with hook portions and brought into contact with the nail from front and back, and hook portions 16 to be hooked to at least one of both sides in the width direction of the nail and a tip portion from the back side, the elastic wire material being made of a superelastic alloy, and the hook portions 16 being hooked such that an end face of the elastic wire material makes contact with the back side of the nail.

## Description

### TECHNICAL FIELD

The present invention relates to an incurvated nail correction tool capable of correcting the deformation of a deformed or ingrown nail.

### BACKGROUND ART

Conventional techniques for correcting the deformation of an incurvated or ingrown nail involved passing a wire through a hole at the tip of the nail, or removing the root of the nail so that the nail stops growing. These methods imposed a heavy burden on the patient, and not without pain.

In contrast to the methods described above that can only be carried out by a doctor, a simple method a patient can take alone is to put on an incurvated nail correction tool such as the one in Patent Literature 1.

### PRIOR ART LITERATURE

### PATENT LITERATURE

[Patent Literature 1] Japanese Utility Model Application Laid-Open No. Hei 4-99914

### SUMMARY OF THE INVENTION

### PROBLEMS TO BE SOLVED BY THE INVENTION

However, with the incurvated nail correction tool of Patent Literature 1, the force applied for correction is small, and it involved pain because of a bar-like end portion being inserted between the nail and nail bed.

The present invention was devised in view of the above problem, and relates to an easy-to-put-on, pain-free incurvated nail correction tool for deformed nails.

### MEANS FOR SOLVING THE PROBLEMS

Through vigorous research, the present inventor has found out that the above problem can be solved by an incurvated nail correction tool including a central portion made of an elastic wire material contacting a nail plate and bent in a waveform within a flat or curved plane, U-shaped portions connecting both ends of this central portion to hook portions and designed to make contact with the nail from front and back, and hook portions to be hooked to at least one of both sides in the width direction of the nail and a tip portion of the nail from the back side of the nail.

In summary, the above-described objectives are achieved by the following embodiment of the present invention.

(1) An incurvated nail correction tool, comprising a central portion capable of being in contact with a nail plate, and a hook portion connected to this central portion to be hooked to at least one of a side in a width direction of a nail and a tip portion of the nail from a back side of the nail, wherein the central portion is made of an elastic wire material, at least one end thereof being connected to the hook portion via a U-shaped portion, and the hook portion is hooked such that an end portion of the elastic wire material makes at least partial contact with the back side of the nail.

(2) An incurvated nail correction tool, comprising a central portion capable of being in contact with a nail plate, and a hook portion connected to this central portion to be hooked to at least one of a side in a width direction of a nail and a tip portion of the nail from a back side of the nail, wherein the central portion is made of an elastic wire material, at least one end thereof being connected to the hook portion via a U-shaped portion, and the hook portion is made of the elastic wire material bent in a circular arc shape and configured to make contact with the back side of the nail when the central portion makes contact with the nail plate.

(3) An incurvated nail correction tool, comprising a central portion capable of being in contact with a nail plate, a hook portion connected to this central portion to be hooked to one of a side in a width direction of a nail and a tip portion of the nail from a back side of the nail, and a bond portion bondable to the nail plate, wherein the central portion is made of an elastic wire material, one end thereof being contained in and secured by the bond portion, and the other end thereof being connected to the hook portion via a U-shaped portion, and the hook portion is hooked such that an end face of the elastic wire material makes contact with the back side of the nail.

(4) An incurvated nail correction tool, comprising a central portion capable of being in contact with a nail plate, a hook portion connected to this central portion to be hooked to one of a side in a width direction of a nail and a tip portion of the nail from a front side of the nail, and a bond portion bondable to the nail plate, wherein the central portion is made of an elastic wire material, one end thereof being contained in and secured by the bond portion, and the other end thereof being connected to the hook portion via a U-shaped portion, and the hook portion is made of the elastic wire material bent in a circular arc shape and configured to make contact with the back side of the nail when the central portion makes contact with the nail plate.

(5) An incurvated nail correction tool, comprising a central portion capable of being in contact with a nail plate, and a bond portion bondable to the nail plate, wherein the central portion is made of an elastic wire material, both ends thereof being contained in and secured by the bond portion, and the bond portion is bondable to the nail plate.

(6) The incurvated nail correction tool according to any one of (1) to (5), wherein the elastic wire material is made of a superelastic alloy and/or a superelastic resin.

(7) The incurvated nail correction tool according to any one of (1) to (6), wherein the central portion is made of the elastic wire material having a length twice or more larger than a length in a width direction of the nail.

(8) The incurvated nail correction tool according to any one of (1) to (7), wherein the central portion is bent at least partially in a waveform within a flat or curved plane.

(9) The incurvated nail correction tool according to any one of (1) to (8), wherein the central portion includes at least one loop shape.

(10) The incurvated nail correction tool according to any one of (1) to (4), (6) to (9), wherein the U-shaped portion is configured to contact a tip portion of the nail from front and back of the nail.

(11) The incurvated nail correction tool according to any one of (3) to (10), wherein the bond portion is made of a thermoplastic resin.

(12) The incurvated nail correction tool according to any one of (1) to (11), wherein the central portion is at least partially contained in an elastic material.

### EFFECTS OF THE INVENTION

According to the present invention, an easy-to-put-on, pain-free, and highly effective incurvated nail correction tool can be provided.

### BRIEF DESCRIPTION OF THE DRAWINGS

FIG. 1 is a plan view showing an incurvated nail correction tool according to a first embodiment of the present invention.
FIG. 2 is a partial enlarged view of the incurvated nail correction tool according to the first embodiment of the present invention.
FIG. 3 is a plan view showing a nail with the incurvated nail correction tool according to the first embodiment of the present invention attached thereon.
FIG. 4 is a plan view showing a nail with the incurvated nail correction tool according to a first embodiment of the present invention attached thereon.
FIG. 5 is a pan view showing an incurvated nail correction tool according to a second embodiment of the present invention.
FIG. 6 is a pan view showing a nail with the incurvated nail correction tool according to the second embodiment of the present invention attached thereon.
FIG. 7 is a pan view showing an incurvated nail correction tool according to a third embodiment of the present invention.
FIG. 8 is a plan view showing a nail with the incurvated nail correction tool according to the third embodiment of the present invention attached thereon.
FIG. 9 is a pan view showing an incurvated nail correction tool according to a fourth embodiment of the present invention.
FIG. 10 is a plan view showing a nail.
FIG. 11 is a plan view showing a nail with the incurvated nail correction tool according to the fourth embodiment of the present invention attached thereon.
FIG. 12 is a plan view showing a nail with the incurvated nail correction tool according to the fourth embodiment of the present invention attached thereon.
FIG. 13 is a plan view showing a nail with the incurvated nail correction tool according to the fourth embodiment of the present invention attached thereon.
FIG. 14 is a plan view showing another configuration for the incurvated nail correction tools according to the first to fourth embodiments of the present invention.
FIG. 15 is a plan view showing another configuration for the incurvated nail correction tools according to the first to fourth embodiments of the present invention.
FIG. 16 is a plan view showing another configuration for the incurvated nail correction tools according to the first to fourth embodiments of the present invention.
FIG. 17 is a plan view showing another configuration for the incurvated nail correction tools according to the first to fourth embodiments of the present invention.
FIG. 18 is a plan view showing another configuration for the incurvated nail correction tools according to the first to fourth embodiments of the present invention.
FIG. 19 is a plan view showing another configuration for the incurvated nail correction tools according to the first to fourth embodiments of the present invention.
FIG. 20 is a plan view showing another configuration for the incurvated nail correction tools according to the first to fourth embodiments of the present invention.
FIG. 21 is a plan view showing another configuration for the incurvated nail correction tools according to the first to fourth embodiments of the present invention.
FIG. 22 is a plan view showing another configuration for the incurvated nail correction tools according to the first to fourth embodiments of the present invention.
FIG. 23 is a plan view showing another configuration for the incurvated nail correction tools according to the first to fourth embodiments of the present invention.
FIG. 24 is a plan view showing another configuration for the incurvated nail correction tools according to the first to fourth embodiments of the present invention.
FIG. 25 is a plan view showing another configuration for the incurvated nail correction tools according to the first to fourth embodiments of the present invention.
FIG. 26 is a plan view showing an incurvated nail correction tool according to a fifth embodiment of the present invention.
FIG. 27 is a plan view showing an incurvated nail correction tool according to the fifth embodiment of the present invention.
FIG. 28 is a plan view showing a nail with the incurvated nail correction tool according to the fifth embodiment of the present invention attached thereon.
FIG. 29 is a plan view showing an incurvated nail correction tool according to a sixth embodiment of the present invention.
FIG. 30 is a plan view showing an incurvated nail correction tool according to the sixth embodiment of the present invention.
FIG. 31 is a plan view showing a nail with the incurvated nail correction tool according to the sixth embodiment of the present invention attached thereon.
FIG. 32 is a plan view showing a nail with the incurvated nail correction tool according to the sixth embodiment of the present invention attached thereon.
FIG. 33 is a plan view showing an incurvated nail correction tool according to the sixth embodiment of the present invention.
FIG. 34 is a plan view showing an incurvated nail correction tool according to a seventh embodiment of the present invention.
FIG. 35 is a plan view showing a nail with the incurvated nail correction tool according to the seventh embodiment of the present invention attached thereon.
FIG. 36 is a plan view showing an incurvated nail correction tool according to the seventh embodiment of the present invention.
FIG. 37 is a plan view showing an incurvated nail correction tool according to another embodiment of the present invention.
FIG. 38 is a photograph showing the conditions of an incurvated nail with an incurvated nail correction tool according to the embodiment of the present invention attached thereon.
FIG. 39 is a photograph showing the conditions of an incurvated nail with an incurvated nail correction tool according to the embodiment of the present invention attached thereon.
FIG. 40 is a photograph showing the conditions of an incurvated nail with an incurvated nail correction tool according to the embodiment of the present invention attached thereon.
FIG. 41 is a photograph showing the conditions of an incurvated nail with an incurvated nail correction tool according to the embodiment of the present invention attached thereon.
FIG. 42 is a photograph showing the conditions of an incurvated nail with an incurvated nail correction tool according to the embodiment of the present invention attached thereon.
FIG. 43 is a photograph showing the conditions of an incurvated nail with an incurvated nail correction tool according to the embodiment of the present invention attached thereon.
FIG. 44 is a photograph showing the conditions of an incurvated nail with an incurvated nail correction tool according to the embodiment of the present invention attached thereon.
FIG. 45 is a photograph showing the conditions of an incurvated nail with an incurvated nail correction tool according to the embodiment of the present invention attached thereon.
FIG. 46 is a photograph showing the conditions of an incurvated nail with an incurvated nail correction tool according to the embodiment of the present invention attached thereon.
FIG. 47 is a photograph showing the conditions of an incurvated nail with an incurvated nail correction tool according to the embodiment of the present invention attached thereon.
FIG. 48 is a photograph showing the conditions of an incurvated nail with an incurvated nail correction tool according to the embodiment of the present invention attached thereon.
FIG. 49 is a photograph showing the conditions of an incurvated nail with an incurvated nail correction tool according to the embodiment of the present invention attached thereon.
FIG. 50 is a photograph showing the conditions of an incurvated nail with an incurvated nail correction tool according to the embodiment of the present invention attached thereon.
FIG. 51 is a photograph showing the conditions of an incurvated nail with an incurvated nail correction tool according to the embodiment of the present invention attached thereon.
FIG. 52 is a photograph showing the conditions of an incurvated nail with an incurvated nail correction tool according to the embodiment of the present invention attached thereon.
FIG. 53 is a photograph showing the conditions of an incurvated nail with an incurvated nail correction tool according to the embodiment of the present invention attached thereon.
FIG. 54 is a photograph showing the conditions of an incurvated nail with an incurvated nail correction tool according to the embodiment of the present invention attached thereon.

### EMBODIMENTS OF THE INVENTION

Embodiments of the present invention will be hereinafter described in detail with reference to the drawings.

### [First Embodiment]

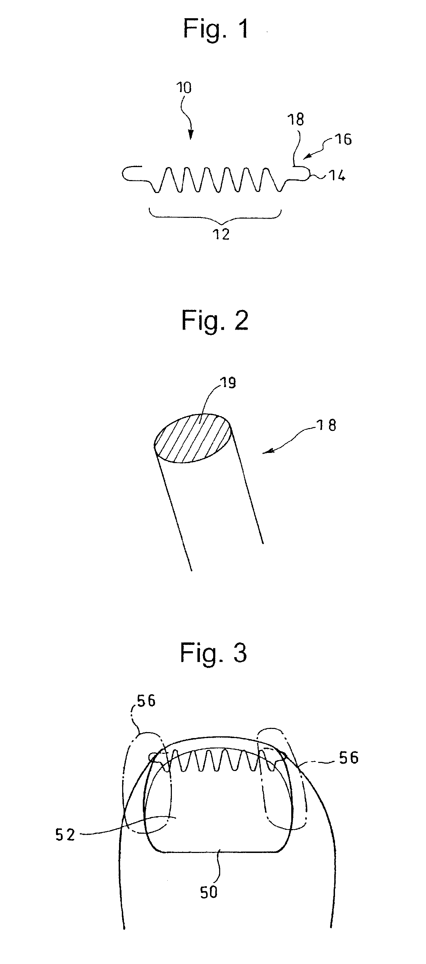

As shown in FIG. 1, the incurvated nail correction tool 10 according to a first embodiment of the present invention is formed by a central portion 12, U-shaped portions 14, and hook portions 16 all made of an elastic wire material in a continuous manner. This elastic wire material is made of a nickel-titanium alloy (nitinol) which is a superelastic alloy. A superelastic alloy herein refers to an alloy that has a recoverable elastic strain of as large as several % to several tens % and shows a generally constant load capacity even when the strain increases.

The central portion 12 is formed by the elastic wire material with a length twice or more larger than the length in the width direction of the nail bent into a waveform within a flat or curved plane, with a substantially rectangular outer shape in plan view, such as to be able to flex elastically as a whole and be in contact with the nail plate, with the number of waves being six.

The central portion 12, in its free state, has its waveform staying within a flat plane, or a curved plane flatter than a curve that runs along the nail plate 52 in contact therewith. Thereby, the central portion 12 can be in contact with the nail plate 52 such that its waveform is within a flat or curved plane along the nail plate 52.

The U-shaped portions 14 are formed by the elastic wire material bent in a substantially U-shape so as to connect both ends of the central portion 12 with the hook portions 16. When the incurvated nail correction tool 10 is attached on a nail 50 such that the waveform forming the central portion 12 is within a flat or curved plane along the nail plate 52, the U-shaped portions 14 can be wound around both side ends 56 of the nail 50 indicated by one-dot chain lines in FIG. 3 to be described later from the nail plate 52 side to the nail bed 54 side to be in contact with these both side ends from the front and the back. The nail plate here refers to a portion of the nail exposed to the outside, while the nail bed refers to a skin portion carrying the nail plate.

The hook portions 16 are formed by the elastic wire material bent continuously from the U-shaped portions 14 such that, when the incurvated nail correction tool 10 is attached on a nail, they are continuous from the U-shaped portions 14 wound around to the back side of the nail 50 and hooked to both sides in the width direction of the nail 50 from the back side of the nail. More specifically, an end portion 18 of the hook portion 16 is bent such that it makes at least partial contact with the back side of a portion at the tip of the nail 50 separated from the nail bed. The end portion 18 has an end face 19 substantially orthogonal to the axis line of the elastic wire member, as shown in FIG. 2. In this way, the end face of the elastic wire material does not enter between the nail 50 and nail bed 54, and since the end portion 18 is blunt, it does not cause pain to the patient.

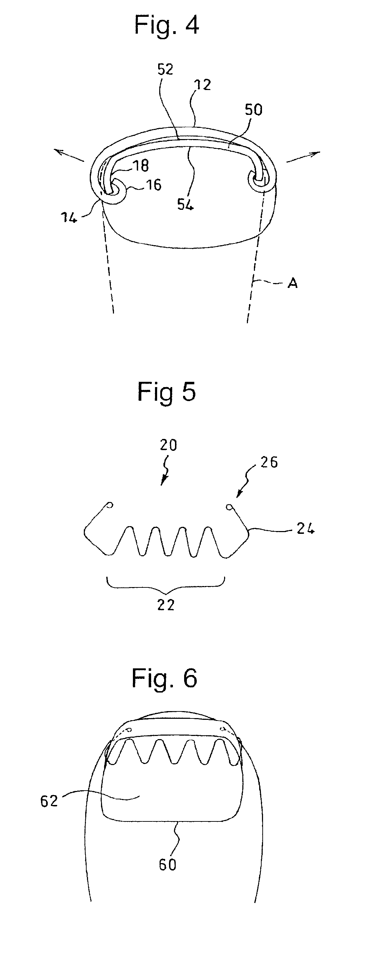

FIG. 3 and FIG. 4 show an example of how the incurvated nail correction tool 10 is attached. FIG. 3 is a diagram of the nail 50 with the incurvated nail correction tool 10 attached thereon viewed from the direction of the nail plate 52, while FIG. 4 is a diagram viewed from the direction of the finger tip. In FIG. 4, the curved plane A along the nail plate 52 is indicated by a broken line. To attach the incurvated nail correction tool 10, with the waveform forming the central portion 12 being within the curved surface A along the nail plate 52, the U-shaped portions 14 at both ends are pressed to flex toward the back side of both ends in the width direction of the nail 50, so that the end portions 18 of the hook portions 16 make contact with the back of the nail 50. In this attached state, the incurvated nail correction tool 10 is freed from the pressing force, whereby a force acts on the tool to return to its initial shape (restoring force) toward the central portion 12 at the center, and this restoring force is transmitted to the hook portions 16 via the U-shaped portions 14. Thereby the hook portions 16 can push up both side ends 56 of the nail 50 in directions of arrows in FIG. 4. At this time, the end face 19 is in contact with the backside of the nail 50 in parallel therewith, so that it can apply the restoring force to the nail 50 without damaging the skin of the nail bed 54.

The incurvated nail correction tool 10 can be attached if there is a portion of about 1 mm of the nail 50 at the tip separated from the nail bed because of its design with the end faces 19 of the hook portions 16 pushing up the nail 50.

The incurvated nail correction tool 10 may be attached on the nail 50 with a tape bonded over the tool so that it is harder to come off.

### [Second Embodiment]

As shown in FIG. 5, the incurvated nail correction tool 20 according to a second embodiment of the present invention is formed by a central portion 22, U-shaped portions 24, and hook portions 26 all made of an elastic wire material in a continuous manner.

The central portion 22 is similar to the central portion 12 of the incurvated nail correction tool 10 of the first embodiment except for the number of waves, which is four.

The U-shaped portions 24 are similar to the U-shaped portions 14 of the incurvated nail correction tool 10 of the first embodiment.

The hook portions 26 are formed by the elastic wire material bent in a circular arc within a plane generally parallel to a flat or curved plane along the nail plate 62 such that, when the incurvated nail correction tool 20 is attached on a nail 60, they are continuous from the U-shaped portions 24 wound around to the back side of the nail 60 and hooked to both sides in the width direction of the nail 60 from the back side of the nail. The hook portions 26 are hooked such that their circular-arc surfaces are in contact with the back side of a portion at the tip of the nail 60 separated from the nail bed, and since they are bent in a circular arc, they do not easily enter between the nail 60 and nail bed even when pressure is applied thereon. The hook portions 26 are oval with a size 3 mm x 2 mm.

FIG. 6 shows an example of how the incurvated nail correction tool 20 is attached. The incurvated nail correction tool 20 can apply a restoring force to the nail 60 more evenly because of the tips of the elastic wire material being bent in a circular arc.

### [Third Embodiment]

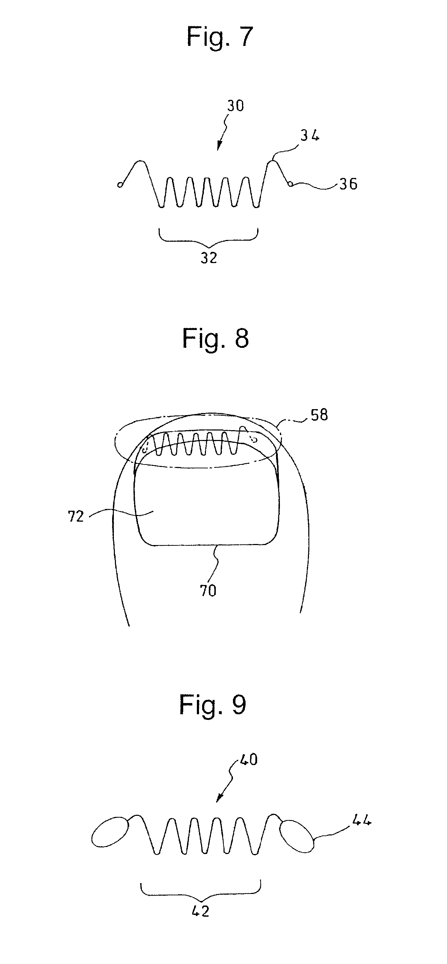

As shown in FIG. 7, the incurvated nail correction tool 30 according to a third embodiment of the present invention is formed by a central portion 32, U-shaped portions 34, and hook portions 36 all made of an elastic wire material in a continuous manner.

The central portion 32 is similar to the central portion 12 of the incurvated nail correction tool 10 of the first embodiment except for the number of waves, which is five.

The U-shaped portions 34 are formed by bending the elastic wire material into a substantially U-shape similarly to the U-shaped portions 14 of the incurvated nail correction tool 10 of the first embodiment, such as to connect both ends of the central portion 32 with the hook portions 36. The U-shaped portions 34 are formed such as to be wound around a tip portion of a nail 70 from the front to the back side thereof and to be in contact with the tip portion from the front and the back, when the incurvated nail correction tool 30 is attached to the nail 70 and the waveform forming the central portion 32 is within the flat or curved plane along the nail plate 72 of the nail 70.

The hook portions 36 are formed by the elastic wire material bent in a circular arc within a plane generally parallel to a flat or curved plane along the nail plate such that, when the incurvated nail correction tool 30 is attached on the nail 70, they are continuous from the ends of the U-shaped portions 34 wound around to the back side of the nail 70 and hooked to the back side of a portion separated from the nail bed of a tip portion 58 of the nail 70, with their circular-arc surfaces being in contact with the back side portion. Since the hook portions 36 are bent in a circular arc, they do not easily enter between the nail 70 and nail bed even when pressure is applied thereon.

FIG. 8 shows an example of how the incurvated nail correction tool 30 is attached. When compared with the incurvated nail correction tool 20 of the second embodiment, the incurvated nail correction tool 30 of the third embodiment has its U-shaped portions in a different position relative to the nail. Since the incurvated nail correction tool 30 is secured to the tip portion 58 of the nail 70 indicated by a one-dot chain line in FIG. 8, its length in the width direction can be easily adjusted.

### [Fourth Embodiment]

As shown in FIG. 7, the incurvated nail correction tool 40 according to a fourth embodiment of the present invention is formed by a central portion 42 made of an elastic wire material and bond portions 44.

The central portion 42 is similar to the central portion 22 of the incurvated nail correction tool 20 of the second embodiment.

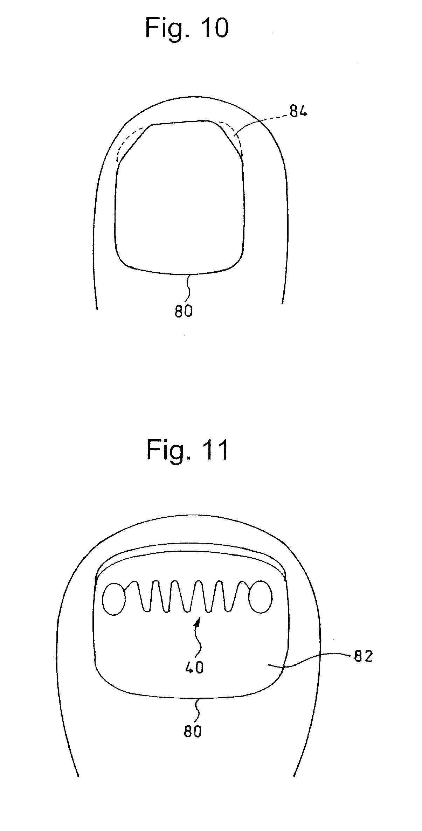

The bond portions 44 are made of a thermoplastic material and designed to be bonded directly or via an adhesive on the nail plate 82 of a nail 80 as shown in FIG. 10. The bond portions 44 are designed to contain and secure the ends of the elastic wire material forming the central portion 42. This thermoplastic material softens at 65°C or higher, and hardens at lower than 65°C. Therefore, the shape of the bond portions 44 can be easily adjusted. When bonding the bond portions 44 on the nail plate 82, they can be bonded more firmly in a softened state, and similarly, when peeling the bond portions 44 from the nail plate 82, they can be peeled off more easily in a softened state.

The incurvated nail correction tool 40 can be used even in cases where incurvated nail correction tools 10, 20, or 30 cannot be used, such as when there is only little portion separated from the nail bed 84 at the tip of the nail 80, or when the tips at both side ends in the width direction of the nail 80 are cut so much that the nail bed 84 is revealed.

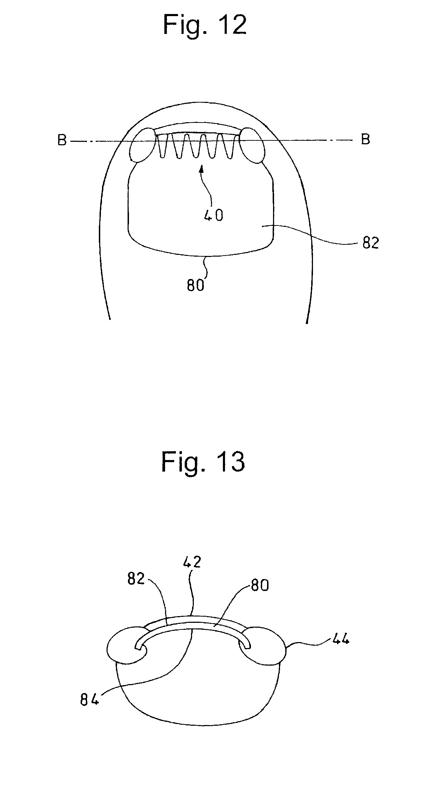

FIG. 11 and FIG. 12 show an example of how the incurvated nail correction tool 40 is attached. A cross sectional view taken along line B-B of FIG. 12 is shown in FIG. 13. As shown in FIG. 13, the bond portions 44 are formed to bridge across the nail plate 82 and nail bed 84, so that the pain caused by the nail 80 that is ingrowing into the nail bed 84 can be alleviated.

Only the bond portions 44 of the incurvated nail correction tool 40 are bonded on the nail plate 82 and the central portion 42 is not bonded. Therefore, the hardening of the adhesive itself does not inhibit the nail correction.

Unlike the incurvated nail correction tools 10 to 30 of the first to third embodiments, the incurvated nail correction tool 40 is designed to apply a restoring force to the nail 80 in such a direction as to lift it up only from the nail plate 82 side. When attaching the incurvated nail correction tool 40 on the nail, heat is applied to the bond portions 44 to soften them so that they are deformed into a shape that conforms to the nail plate 82 and bonded thereto.

Since the bond portions 44 of the incurvated nail correction tool 40 are made of a thermoplastic material, they can be deformed into a shape that conforms to the nail plate 82 and nail bed 84 by heating, and thus the incurvated nail correction tool 40 can have enhanced bondability to the nail plate 82.

The thermoplastic material of the bond portions 44 is replaceable so that the bondability of the bond portions 44 can be maintained at more than a certain level.

The number of waves in the central portion is not limited, but with four or more waves, the incurvated nail correction tool can maintain its elasticity for a long period of time. The incurvated nail correction tool is capable of applying a force in a direction in which the incurvated nail is corrected by this elasticity over a long period of time. There is no upper limit to the number of waves. However, in view of its nature as a tool attached to a nail, a realistic number of waves would be up to about 10. The length in the amplitude direction of the waves of the central portion is not limited, but it will be more effective with the length being about 3 to 4 mm.

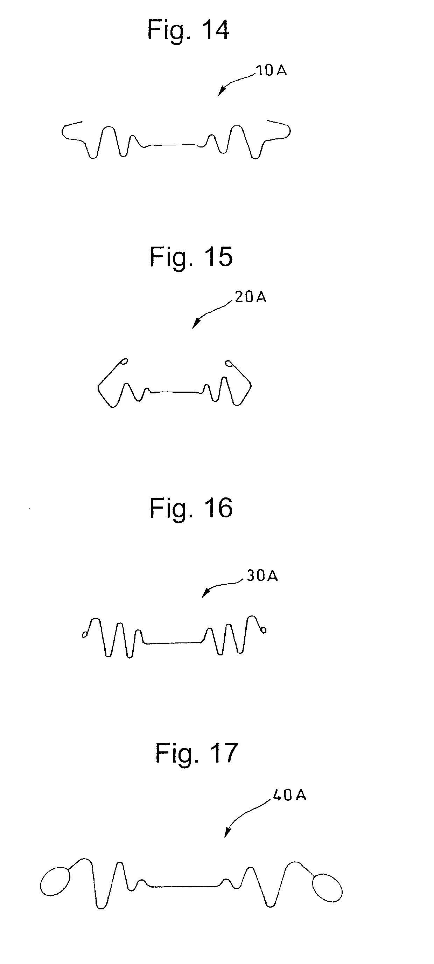

Waves of the central portion may be formed, for example, only at both ends, or only at one end, or only between both ends. The length and amplitude of the waves need not be constant. FIG. 14 shows an incurvated nail correction tool 10A with the central portion being substantially straight in the middle. The incurvated nail correction tool 10A is configured similarly to the incurvated nail correction tool 10 of the first embodiment except for the central portion. In this way, the incurvated nail correction tool 10A can stretch along the longitudinal axis direction and can be attached on the nail while being stretched in the longitudinal axis direction, whereby the incurvated nail correction tool 10A can be secured to the nail more stably. Such an effect can also be achieved if the central portion is curved with a small radius in the middle.

In this way, too, the tool can be formed into a shape more suitable for correction of an incurvated nail sharply curled only at both sides.

Similarly, FIG. 15 shows an incurvated nail correction tool 20A configured similarly to the incurvated nail correction tool 20 of the second embodiment except for the central portion, FIG. 16 shows an incurvated nail correction tool 30A configured similarly to the incurvated nail correction tool 30 of the third embodiment except for the central portion, and FIG. 17 shows an incurvated nail correction tool 40A configured similarly to the incurvated nail correction tool 40 of the fourth embodiment except for the central portion.

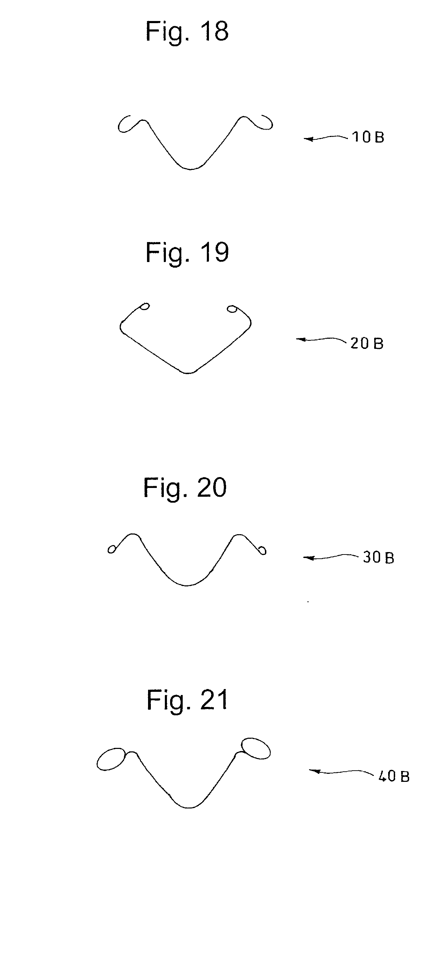

Further, the central portion may have just one wave. FIG. 18 shows an incurvated nail correction tool 10B with one wave in the central portion. The incurvated nail correction tool 10B is configured similarly to the incurvated nail correction tool 10 of the first embodiment except for the central portion.

Similarly, FIG. 19 shows an incurvated nail correction tool 20B configured similarly to the incurvated nail correction tool 20 of the second embodiment except for the central portion, FIG. 20 shows an incurvated nail correction tool 30B configured similarly to the incurvated nail correction tool 30 of the third embodiment except for the central portion, and FIG. 21 shows an incurvated nail correction tool 40B configured similarly to the incurvated nail correction tool 40 of the fourth embodiment except for the central portion.

### [Fifth Embodiment]

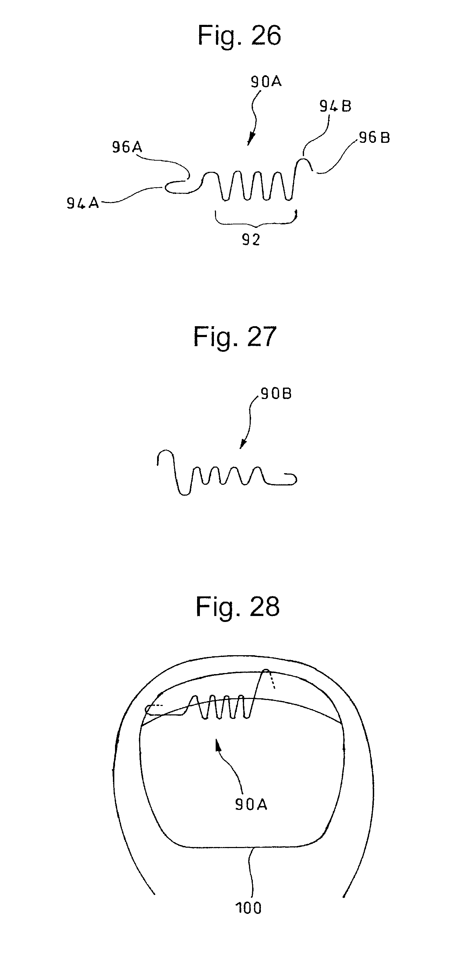

As shown in FIG. 26, the incurvated nail correction tool 90A according to a fifth embodiment of the present invention is formed by a central portion 92, U-shaped portions 94A and 94B, and hook portions 96A and 96B made of an elastic wire material in a continuous manner.

The central portion 92, the U-shaped portion 94A, and the hook portion 96A are similar to the central portion 12, the U-shaped portions 14, and the hook portions 16 of the incurvated nail correction tool 10 of the first embodiment, while the U-shaped portion 94B is similar to the U-shaped portions 34 of the incurvated nail correction tool 30 of the third embodiment.

The hook portion 96B is similar to the hook portion of the incurvated nail correction tool 10 of the first embodiment, and designed to be hooked to a tip portion of a nail 100 from the back side of the nail 100.

The incurvated nail correction tool 90B shown in FIG. 27 is a laterally reversed image of the incurvated nail correction tool 90A.

FIG. 28 shows an example of how the incurvated nail correction tool 90A is attached. Since the incurvated nail correction tool 90A need not be strictly matched with the length in the width direction of the nail, it can be easily adjusted in its length in the width direction.

### [Sixth Embodiment]

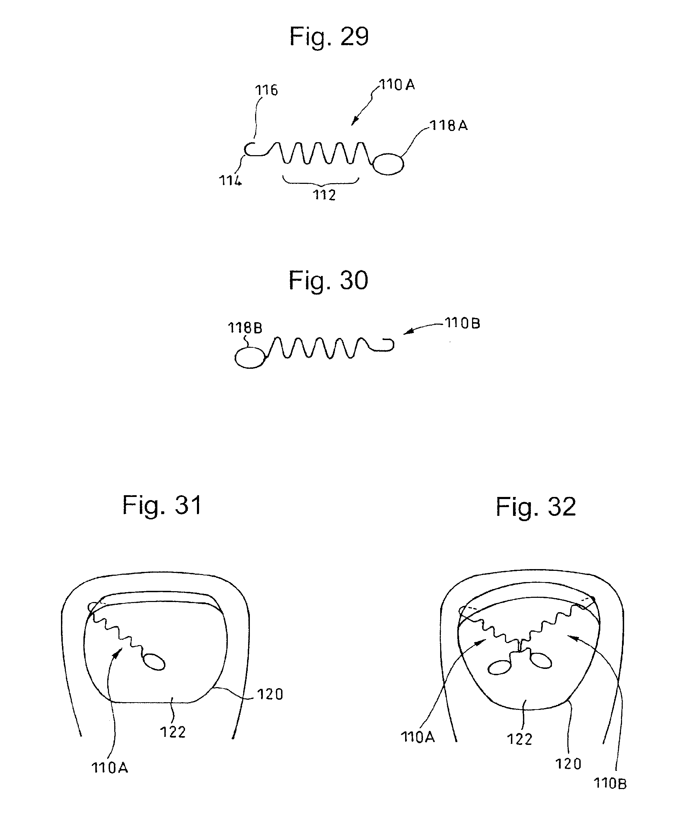

As shown in FIG. 29, the incurvated nail correction tool 110A according to a sixth embodiment of the present invention is formed by a central portion 112, a U-shaped portion 114, and a hook portion 116 made of an elastic wire material, the latter two being continuous from one end of the central portion 112, and a bond portion 118A formed at the other end of the central portion 112.

The central portion 112, the U-shaped portion 114, and the hook portion 116 are similar to the central portion 12, the U-shaped portions 14, and the hook portions 16 of the incurvated nail correction tool 10 of the first embodiment, while the bond portion 118A is similar to the bond portions 44 of the incurvated nail correction tool 40 of the fourth embodiment.

The incurvated nail correction tool 110B shown in FIG. 30 is a laterally reversed image of the incurvated nail correction tool 110A.

FIG. 31 shows an example of how the incurvated nail correction tool 110A is attached, and FIG. 32 shows an example of how the incurvated nail correction tools 110A and 110B are attached. Since the incurvated nail correction tools 110A and 110B are bondable on the nail plate 122 of a nail 120 at respective bond portions 118A and 118B, they are suited for correction of a severely deformed incurvated nail.

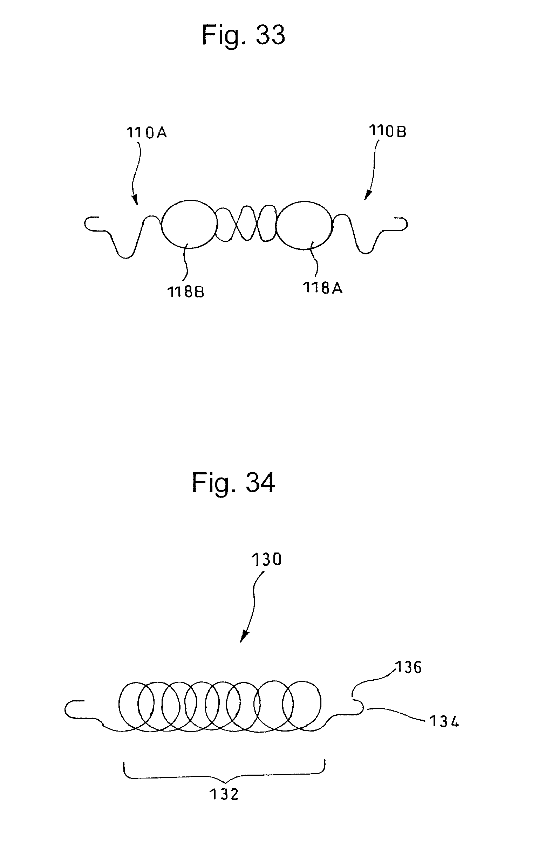

The incurvated nail correction tools 110A and 110B may be combined as shown in FIG. 33 such that the incurvated nail correction tool 110A is secured by the bond portion 118B of the incurvated nail correction tool 110B and the incurvated nail correction tool 110B is secured by the bond portion 118A of the incurvated nail correction tool 110A, so that they can be used as one incurvated nail correction tool.

### [Seventh Embodiment]

As shown in FIG. 34, the incurvated nail correction tool 130 according to a seventh embodiment of the present invention is formed by a central portion 132, U-shaped portions 134, and hook portions 136 made of an elastic wire material in a continuous manner.

The central portion 132 is bent such as to have eight loops within a flat or curved plane, has a substantially rectangular outer shape in plan view, and can be flexed elastically as a whole and be in contact with a nail plate.

The U-shaped portions 134 and the hook portions 136 are similar to the U-shaped portions 14 and the hook portions 16 of the incurvated nail correction tool 10 of the first embodiment, respectively.

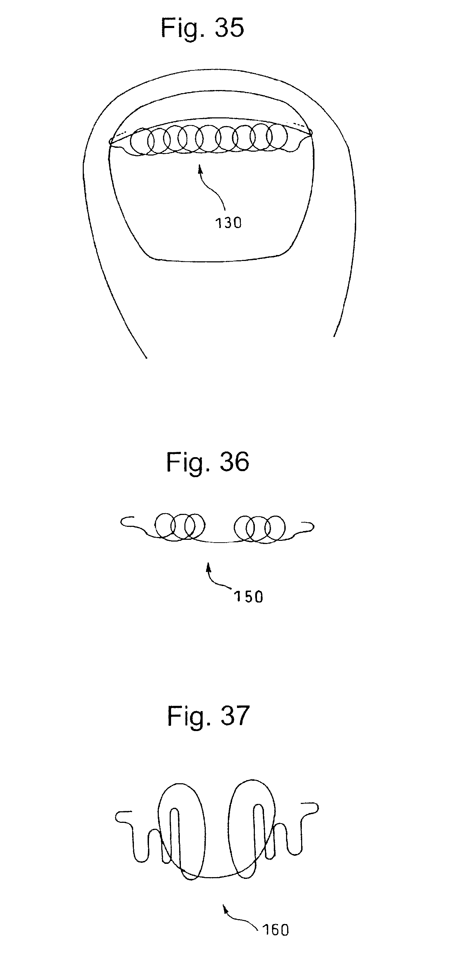

FIG. 35 shows an example of how the incurvated nail correction tool 130 is attached. Since the incurvated nail correction tool 130 provides sufficient elasticity even though it is short in the width direction, it can advantageously be used for incurvated nails of fingers, in particular, other than the thumb.

### [Eighth Embodiment]

The incurvated nail correction tool according to an eighth embodiment of the present invention has the same shape as the incurvated nail correction tool 10 of the first embodiment, but the elastic wire material forming the central portion, U-shaped portions, and hook portions is made of polyphenylsulfone, which is a superelastic resin. A superelastic resin here refers to a resin that has a recoverable elastic strain of as large as several % to several tens % and shows a generally constant load capacity even when the strain increases.

The incurvated nail correction tools according to the first to sixth embodiments of the present invention include a wavy shape in at least part of the central portion, and therefore exhibit high elasticity in a direction perpendicular to the nail plate and can be easily attached on the nail. They can be stably secured on the nail, and provide good wear comfort. With the use of the elastic wire material made of a superelastic alloy, they can provide a suitable restoring force, without applying stress on the nail, and are unlikely to damage the nail and nail bed.

If the central portion has a straight shape, a large force will be applied to both side ends of the nail, so that the tool is hard to put on and can damage the nail. For this reason, the incurvated nail correction tool of the present invention cannot have a central portion of a straight shape.

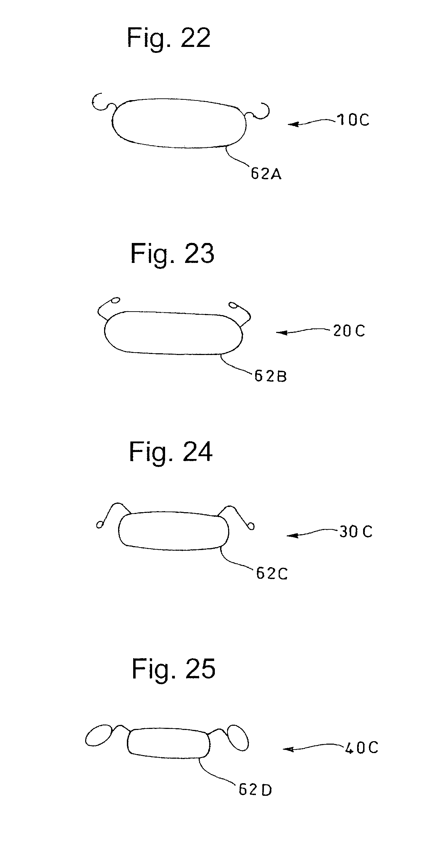

The central portion may be contained, at least partially, in an elastic material. Thereby, the wear comfort of the incurvated nail correction tool can further be improved. FIG. 22 shows an incurvated nail correction tool 10C with an elastic material 62A containing the central portion. The incurvated nail correction tool 10C is configured similarly to the incurvated nail correction tool 10 of the first embodiment except for the central portion.

Similarly, FIG. 23 shows an incurvated nail correction tool 20C configured similarly to the incurvated nail correction tool 20 of the second embodiment except for the central portion contained in an elastic material 62B, FIG. 24 shows an incurvated nail correction tool 30C configured similarly to the incurvated nail correction tool 30 of the third embodiment except for the central portion contained in an elastic material 62C, and FIG. 25 shows an incurvated nail correction tool 40C configured similarly to the incurvated nail correction tool 40 of the fourth embodiment except for the central portion contained in an elastic material 62D. For the elastic material, polymer materials such as plastics, silicone resins, silicone rubbers, and the like may be used.

The U-shaped portions are made as thick as or thicker than the nail.

The size of the hook portions is not limited, but it would be more effective if they are of the size that does not damage the nail and nail bed and allows them to be hooked stably on the nail.

According to the embodiments of the present invention, temperature adjustment as with an incurvated nail correction tool or the like using a shape-memory alloy is not necessary and the tool need only be attached on a nail, so that the patient can easily correct an incurvated nail alone.

The elastic wire material forming the incurvated nail correction tool of the present invention may be one of superelastic alloys other than nitinol, including, for example, a copper based alloy such as Cu-Mn-Al alloy, Fe-Al based alloy, Ti-Nb-Al based alloy or the like. The elastic wire material may also be made of superelastic resins other than polyphenylsulfone, including, for example, natural rubber, synthetic rubber, polyisoprene, styrene-butadiene copolymer, polyethylene, chlorinated polyethylene, fluorine resins,
esters of polyacrylic acid, esters of polymethacrylic acid, polysiloxane, silicone resins, polyvinyl chloride, polyurethane, polyimide, polyamide, polysilane, or the like.

Further, the elastic wire material may be made of a material other than a superelastic alloy or a superelastic resin, or may be made of a suitable combination of a superelastic alloy, superelastic resin, and other materials.

The bond portion 44 of the incurvated nail correction tool 40 of the fourth embodiment is not limited to a thermoplastic material and may be made of other thermoplastic resin materials. Adhesive materials other than thermoplastic resin materials may also be used, or, non-adhesive materials may be used so that the bond portion is bonded to the nail plate with an adhesive. The bond portion may be bonded only to the nail plate without touching the nail bed.

The thickness of the elastic wire material used for the incurvated nail correction tools of embodiments of the present invention is not limited, but may be 0.5 mm to 0.8 mm to provide a sufficient effect. Wire thinner than 0.5 mm may not provide a sufficient restoring force and not be able to correct an incurvated nail. Wire thicker than 0.8 mm may generate too large a restoring force and cause too much stress on the nail.

If the number of waves is three, then a 0.5 mm or 0.6 mm wire may be used and the waves in the central portion may have a length of about 6 mm in the amplitude direction. If the number of waves is two, then a 0.5 mm wire may be used and the waves in the central portion may have a length of about 8 mm in the amplitude direction.

The hook portions may be in contact with the back side of the nail of at least one of both sides in the width direction of the nail and the tip portion. They may be in contact with points where these two meet, i.e., corners of the nail.

The hook portions 26 and 36 of the incurvated nail correction tools 20 and 30 of the second and third embodiments may be bent in a circular arc shape within a plane other than the plane substantially parallel to a flat or curved plane along the nail plate. That is, parts other than the circular-arc surfaces, for example, side portions of the circular-arc surfaces may be brought into contact with and hooked to the back side of the portion at the tip of the nail separated from the nail bed.

While the incurvated nail correction tool 130 of the seventh embodiment has eight loops, the incurvated nail correction tool of the present invention may have at least one loop. For example, as shown in FIG. 36, it may be configured as an incurvated nail correction tool 150 having six loops.

The incurvated nail correction tool according to embodiments of the present invention may be used not only for correction of an incurvated nail, but also for prevention of nail deformation.

The central portion of the incurvated nail correction tool of the present invention may have other shapes other than the wavy shapes or loop shapes as shown in the embodiments.
For example, as shown in FIG. 37, it may be configured as an incurvated nail correction tool 160 having a combination of a wavy shape and a loop shape.

In actual use of the incurvated nail correction tool of the present invention, a most suitable one of various forms as has been shown in the first to seventh embodiments may be selected depending on the shape of the nail on which it is to be attached.

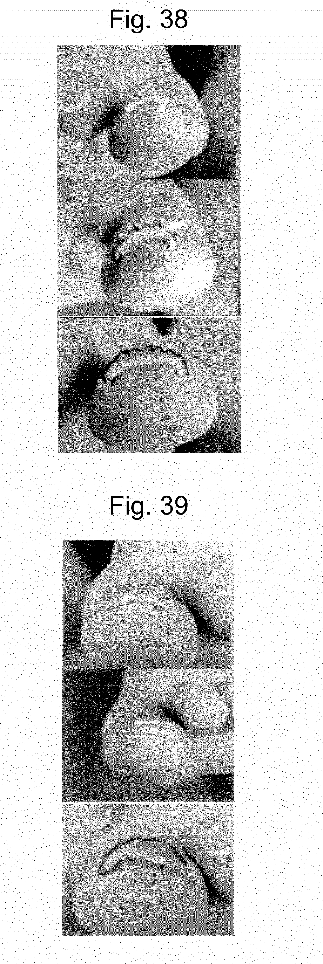

FIG. 38 to FIG. 54 show how an incurvated nail is actually corrected using the incurvated nail correction tools according to embodiments of the present invention.

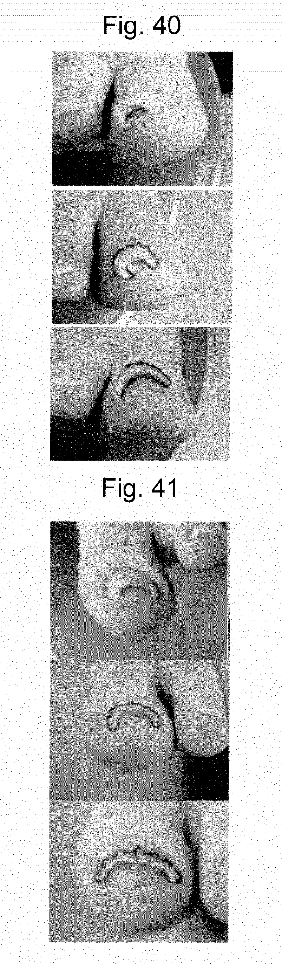

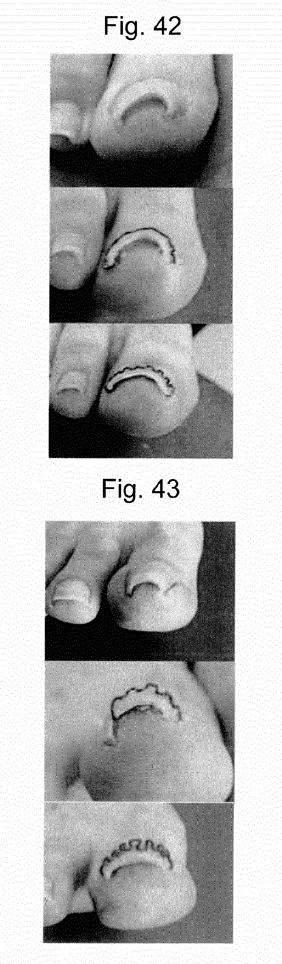

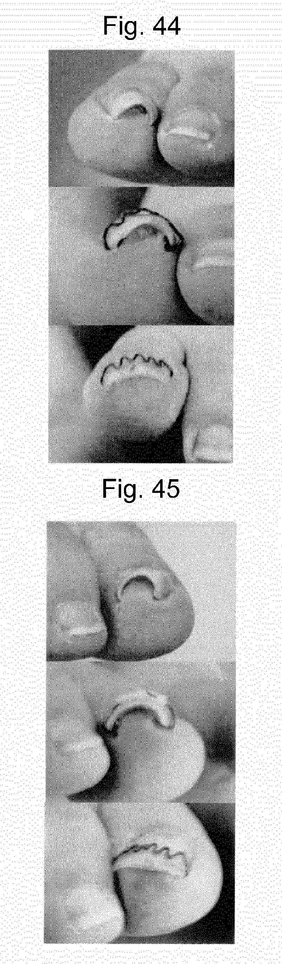

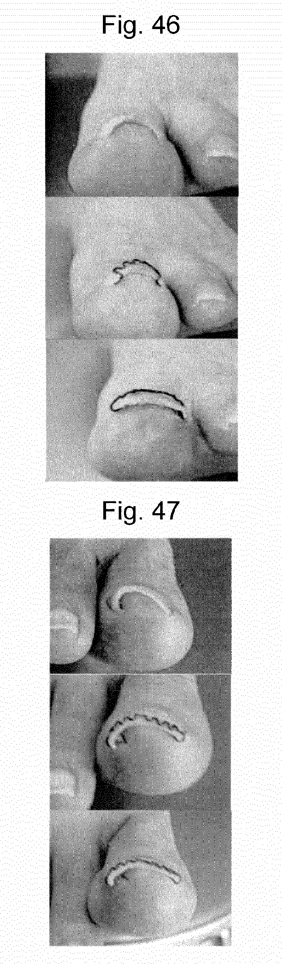

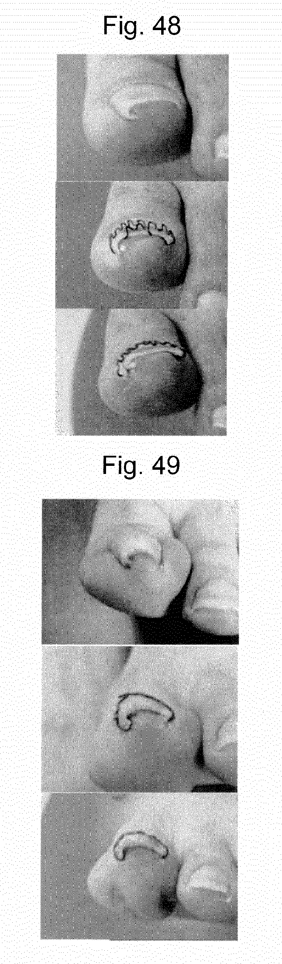

FIG. 38 shows, from top to bottom, the states of an incurvated nail before attachment of the incurvated nail correction tool 20 of the second embodiment, on the first day it was attached, and on the 22nd day since it was attached. FIG. 39 shows, from top to bottom, the states of an incurvated nail before attachment of the incurvated nail correction tool 10 of the first embodiment, on the first day it was attached, and on the 14th day since it was attached. FIG. 40 shows, from top to bottom, the states of an incurvated nail before attachment of the incurvated nail correction tool 20 of the second embodiment, on the first day it was attached, and on the 12th day since it was attached. FIG. 41 shows, from top to bottom, the states of an incurvated nail before attachment of the incurvated nail correction tool 10 of the first embodiment, on the first day it was attached, and on the 38th day since it was attached. FIG. 42 shows the states of another incurvated nail similar to that of FIG. 41. FIG. 43 shows, from top to bottom, the states of an incurvated nail before attachment of the incurvated nail correction tool 90A of the fifth embodiment, on the first day it was attached, and on the 14th day since it was attached. FIG. 44 shows, from top to bottom, the states of an incurvated nail before attachment of the incurvated nail correction tool 20 of the second embodiment,
on the first day it was attached, and on the 83rd day since it was attached. FIG. 45 shows the states of another incurvated nail similar to that of FIG. 44. FIG. 46 shows, from top to bottom, the states of an incurvated nail before attachment of the incurvated nail correction tool 30 of the third embodiment, on the first day it was attached, and on the 3rd day since it was attached when the incurvated nail correction tool 30 was removed and then the incurvated nail correction tool 10 of the first embodiment was attached. FIG. 47 shows, from top to bottom, the states of an incurvated nail before attachment of the incurvated nail correction tool 10 of the first embodiment, on the first day it was attached, and on the second day since it was attached. FIG. 48 shows, from top to bottom, the states of an incurvated nail before attachment of the incurvated nail correction tools 90A, 90B of the fifth embodiment, on the first day it was attached, and on the second day since it was attached when the incurvated nail correction tools 90A, 90B were removed and then the incurvated nail correction tool 10 of the first embodiment was attached. FIG. 49 shows, from top to bottom, the states of an incurvated nail before attachment of the incurvated nail correction tool 10 of the first embodiment, on the first day it was attached, and on the 14th day since it was attached.

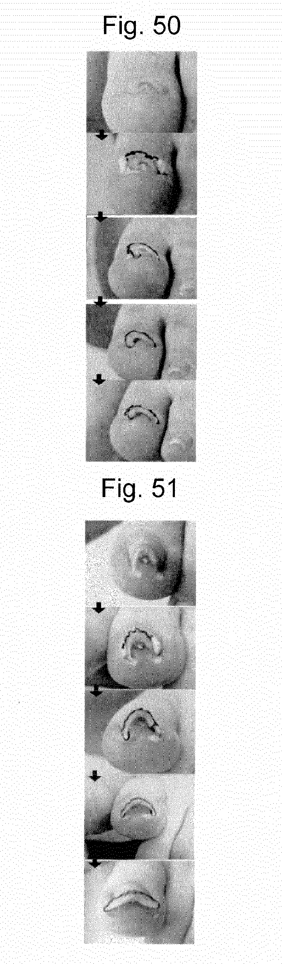

FIG. 38 to FIG. 49 all show that the incurvated nail has been corrected by the incurvated nail correction tool. It can also be seen that the elasticity of the incurvated nail correction tool has been maintained over time. Plural types of incurvated nail correction tools may be used to correct one incurvated nail, as shown in FIG. 32 or FIG. 34. FIG. 50 to FIG. 52 show examples of use of plural types of incurvated nail correction tools.

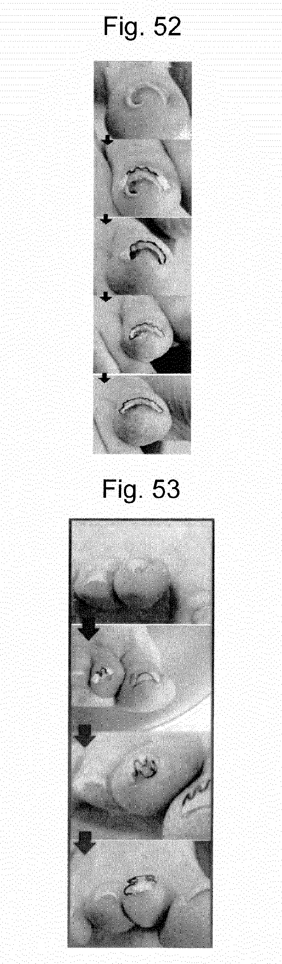

FIG. 50 shows, from top to bottom, the states of an incurvated nail before attachment of the incurvated nail correction tool 40 of the fourth embodiment, on the first day it was attached, on the 32nd day since it was attached when the incurvated nail correction tool 40 was removed and then the incurvated nail correction tool 10 of the first embodiment was attached, and on the 90th day and 104th day since the incurvated nail correction 40 was attached. FIG. 51 shows, from top to bottom, the states of an incurvated nail before attachment of the incurvated nail correction tool 40 of the fourth embodiment, on the first day it was attached, on the 20th day since it was attached when the incurvated nail correction tool 40 was removed and then the incurvated nail correction tool 90B of the fifth embodiment was attached, on the 27th day since the incurvated nail correction tool 40 was attached when the incurvated nail correction tool 90B was removed and then the incurvated nail correction tool 10 of the first embodiment was attached, and on the 48th day since the incurvated nail correction tool 40 was attached. FIG. 52 shows the states of another incurvated nail similar to that of FIG. 51.

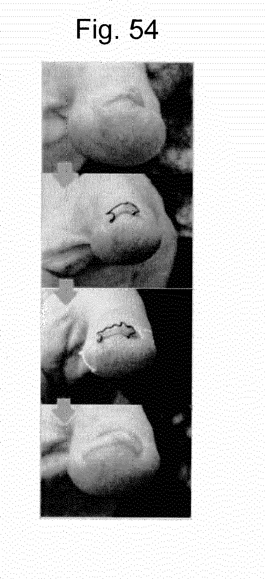

FIG. 53 and FIG. 54 show how an incurvated nail, which is so small that it could not be corrected with a conventional incurvated nail correction tool, is corrected.

FIG. 53 shows, from top to bottom, the states of an incurvated nail before attachment of the incurvated nail correction tool 40 of the fourth embodiment, on the first day it was attached, on the 25th day since it was attached when the incurvated nail correction tool 40 was removed and then the incurvated nail correction tool 90A of the fifth embodiment was attached, and the 87th day since the incurvated nail correction tool 40 was attached when the incurvated nail correction tool 90A was removed and then the incurvated nail correction tool 150 of the 7th embodiment was attached. FIG. 54 shows, from top to bottom, the states of an incurvated nail before attachment of the incurvated nail correction tool 30 of the third embodiment, on the first day it was attached, on the 9th day since it was attached and on the 70th day since it was attached when the incurvated nail correction tool 30 was removed.

As shown in FIG. 53 and FIG. 54, with the incurvated nail correction tool according to embodiments of the present invention, even an incurvated nail which is so small that it could not be corrected with a conventional incurvated nail correction tool can be corrected.

### INDUSTRIAL APPLICABILITY

The incurvated nail correction tool according to the present invention is easy to put on without causing any pain, and therefore can widely be applied to incurvated nails of various shapes.

10, 10A, 10B, 10C, 20, 20A, 20B, 20C, 30, 30A, 30B, 30C, 40, 40A, 40B, 40C, 90A, 90B, 110A, 110B, 130, 150, 160···Incurvated nail correction tool
12, 22, 32, 42, 92, 112, 132···Central portion
14, 24, 34, 94A, 94B, 114, 134···U-shaped portion
16, 26, 36, 96A, 96B,116, 136···Hook Portion
18 ... End portion
44, 118A, 118B···Bond portion
50, 60, 70, 80, 100, 120···Nail
52, 62, 72, 82, 122···Nail plate
54, 84···Nail bed
56···Both side ends
58···Tip portion
62A, 62B, 62C, 62D···Elastic material

## Claims

1. An incurvated nail correction tool, comprising a central portion capable of being in contact with a nail plate, and a hook portion connected to this central portion to be hooked to at least one of a side in a width direction of a nail and a tip portion of the nail from a back side of the nail, wherein
the central portion is made of an elastic wire material, at least one end thereof being connected to the hook portion via a U-shaped portion, and
the hook portion is hooked such that an end portion of the elastic wire material makes at least partial contact with the back side of the nail.

2. An incurvated nail correction tool, comprising a central portion capable of being in contact with a nail plate, and a hook portion connected to this central portion to be hooked to at least one of a side in a width direction of a nail and a tip portion of the nail from a back side of the nail, wherein
the central portion is made of an elastic wire material, at least one end thereof being connected to the hook portion via a U-shaped portion, and
the hook portion is made of the elastic wire material bent in a circular arc shape and configured to make contact with the back side of the nail when the central portion makes contact with the nail plate.

3. An incurvated nail correction tool, comprising a central portion capable of being in contact with a nail plate, a hook portion connected to this central portion to be hooked to one of a side in a width direction of a nail and a tip portion of the nail from a back side of the nail, and a bond portion bondable to the nail plate, wherein
the central portion is made of an elastic wire material, one end thereof being contained in and secured by the bond portion, and the other end thereof being connected to the hook portion via a U-shaped portion, and
the hook portion is hooked such that an end face of the elastic wire material makes contact with the back side of the nail.

4. An incurvated nail correction tool, comprising a central portion capable of being in contact with a nail plate, a hook portion connected to this central portion to be hooked to one of a side in a width direction of a nail and a tip portion of the nail from a front side of the nail, and a bond portion bondable to the nail plate, wherein
the central portion is made of an elastic wire material, one end thereof being contained in and secured by the bond portion, and the other end thereof being connected to the hook portion via a U-shaped portion, and
the hook portion is made of the elastic wire material bent in a circular arc shape and configured to make contact with the back side of the nail when the central portion makes contact with the nail plate.

5. An incurvated nail correction tool, comprising a central portion capable of being in contact with a nail plate, and a bond portion bondable to the nail plate, wherein
the central portion is made of an elastic wire material, both ends thereof being contained in and secured by the bond portion, and
the bond portion is bondable to the nail plate.

6. The incurvated nail correction tool according to any one of claims 1 to 5, wherein the elastic wire material is made of a superelastic alloy and/or a superelastic resin.

7. The incurvated nail correction tool according to any one of claims 1 to 5, wherein the central portion is made of the elastic wire material having a length twice or more larger than a length in a width direction of the nail.

8. The incurvated nail correction tool according to claim 6, wherein the central portion is made of the elastic wire material having a length twice or more larger than a length in a width direction of the nail.

9. The incurvated nail correction tool according to any one of claims 1 to 5, wherein the central portion is bent at least partially in a waveform within a flat or curved plane.

10. The incurvated nail correction tool according to claim 6, wherein the central portion is bent at least partially in a waveform within a flat or curved plane.

11. The incurvated nail correction tool according to claim 7, wherein the central portion is bent at least partially in a waveform within a flat or curved plane.

12. The incurvated nail correction tool according to claim 8, wherein the central portion is bent at least partially in a waveform within a flat or curved plane.

13. The incurvated nail correction tool according to any one of claims 1 to 5, wherein the central portion includes at least one loop shape.

14. The incurvated nail correction tool according to claim 6, wherein the central portion includes at least one loop shape.

15. The incurvated nail correction tool according to any one of claims 1 to 4, wherein the U-shaped portion is configured to contact a tip portion of the nail from front and back of the nail.

16. The incurvated nail correction tool according to claim 6, wherein the U-shaped portion is configured to contact a tip portion of the nail from front and back of the nail.

17. The incurvated nail correction tool according to any one of claims 3 to 5, wherein the bond portion is made of a thermoplastic resin.

18. The incurvated nail correction tool according to claim 6, wherein the bond portion is made of a thermoplastic resin.

19. The incurvated nail correction tool according to any one of claims 1 to 5, wherein the central portion is at least partially contained in an elastic material.

20. The incurvated nail correction tool according to claim 6, wherein the central portion is at least partially contained in an elastic material.

## Amended claims

### Amended claims under Art. 19.1 PCT

1. (Amended) An incurvated nail correction tool, comprising a central portion capable of being in contact with a nail plate, and a hook portion connected to this central portion to be hooked to at least one of a side in a width direction of a nail and a tip portion of the nail from a back side of the nail, wherein
the central portion is made of an elastic wire material, bent at least partially in a waveform within a flat or curved plane, and at least one end thereof being connected to the hook portion via a U-shaped portion, and
the hook portion is hooked such that an end portion of the elastic wire material makes at least partial contact with the back side of the nail.

2. (Amended) An incurvated nail correction tool, comprising a central portion capable of being in contact with a nail plate, and a hook portion connected to this central portion to be hooked to at least one of a side in a width direction of a nail and a tip portion of the nail from a back side of the nail, wherein
the central portion is made of an elastic wire material, bent at least partially in a waveform within a flat or curved plane, and at least one end thereof being connected to the hook portion via a U-shaped portion, and
the hook portion is made of the elastic wire material bent in a circular arc shape and configured to make contact with the back side of the nail when the central portion makes contact with the nail plate.

3. (Amended) An incurvated nail correction tool, comprising a central portion capable of being in contact with a nail plate, a hook portion connected to this central portion to be hooked to one of a side in a width direction of a nail and a tip portion of the nail from a back side of the nail, and a bond portion bondable to the nail plate, wherein
the central portion is made of an elastic wire material, bent at least partially in a waveform within a flat or curved plane, and one end thereof being contained in and secured by the bond portion, and the other end thereof being connected to the hook portion via a U-shaped portion, and
the hook portion is hooked such that an end face of the elastic wire material makes contact with the back side of the nail.

4. (Amended) An incurvated nail correction tool, comprising a central portion capable of being in contact with a nail plate, a hook portion connected to this central portion to be hooked to one of a side in a width direction of a nail and a tip portion of the nail from a front side of the nail, and a bond portion bondable to the nail plate, wherein
the central portion is made of an elastic wire material, bent at least partially in a waveform within a flat or curved plane, and one end thereof being contained in and secured by the bond portion, and the other end thereof being connected to the hook portion via a U-shaped portion, and
the hook portion is made of the elastic wire material bent in a circular arc shape and configured to make contact with the back side of the nail when the central portion makes contact with the nail plate.

5. (Amended) An incurvated nail correction tool, comprising a central portion capable of being in contact with a nail plate, and a bond portion bondable to the nail plate, wherein
the central portion is made of an elastic wire material, bent at least partially in a waveform within a flat or curved plane, and both ends thereof being contained in and secured by the bond portion, and
the bond portion is bondable to the nail plate.

6. The incurvated nail correction tool according to any one of claims 1 to 5, wherein the elastic wire material is made of a superelastic alloy and/or a superelastic resin.

7. The incurvated nail correction tool according to any one of claims 1 to 5, wherein the central portion is made of the elastic wire material having a length twice or more larger than a length in a width direction of the nail.

8. The incurvated nail correction tool according to claim 6, wherein the central portion is made of the elastic wire material having a length twice or more larger than a length in a width direction of the nail.

9. (Canceled)

10. (Canceled)

11. (Canceled)

12. (Canceled)

13. The incurvated nail correction tool according to any one of claims 1 to 5, wherein the central portion includes at least one loop shape.

14. The incurvated nail correction tool according to claim 6, wherein the central portion includes at least one loop shape.

15. The incurvated nail correction tool according to any one of claims 1 to 4, wherein the U-shaped portion is configured to contact a tip portion of the nail from front and back of the nail.

16. The incurvated nail correction tool according to claim 6, wherein the U-shaped portion is configured to contact a tip portion of the nail from front and back of the nail.

17. The incurvated nail correction tool according to any one of claims 3 to 5, wherein the bond portion is made of a thermoplastic resin.

18. The incurvated nail correction tool according to claim 6, wherein the bond portion is made of a thermoplastic resin.

19. The incurvated nail correction tool according to any one of claims 1 to 5, wherein the central portion is at least partially contained in an elastic material.

20. The incurvated nail correction tool according to claim 6, wherein the central portion is at least partially contained in an elastic material.
